(19) Europäisches Patentamt
European Patent Office
Office européen des brevets

(11) Publication number: 0 095 152
B1

(12) EUROPEAN PATENT SPECIFICATION

(45) Date of publication of patent specification: 22.10.86

(21) Application number: 83104984.6

(22) Date of filing: 19.05.83

(51) Int. Cl.[4]: C 07 D 213/50,
C 07 D 213/85, C 07 D 213/82
// A61K31/44

(54) **A process for preparing nicotinonitriles and intermediates used therein.**

(30) Priority: 24.05.82 US 381162
03.03.83 US 471961
24.05.82 US 381062

(43) Date of publication of application:
30.11.83 Bulletin 83/48

(45) Publication of the grant of the patent:
22.10.86 Bulletin 86/43

(84) Designated Contracting States:
AT BE CH DE FR GB IT LI NL SE

(56) References cited:
GB-A-2 070 008
US-A-2 824 121
US-A-4 137 233
US-A-4 223 149
US-A-4 262 000
US-A-4 276 293
US-A-4 313 951
US-A-4 347 363

(73) Proprietor: STERLING DRUG INC.
90 Park Avenue
New York New York (US)

(72) Inventor: Gelotte, Karl Olaf
R.D. 1
East Nassau New York (US)
Inventor: Singh, Baldev
3 Blue Mountain Trail
East Greenbush New York (US)

(74) Representative: Baillie, Iain Cameron et al
c/o Ladas & Parry Isartorplatz 5
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 152 B1

**Description**

This invention relates to a process for preparing cadiotonically active 1,2-dihydro-6-(lower-alkyl)-2-oxo-5-(pyridinyl)nicotinonitriles and to intermediates used therein.

U.S. Patent 4,313,951 discloses *inter alia*, the process for preparing a 1,2-dihydro-6-(lower-alkyl)-2-oxo-5-(pyridinyl)nicotinonitrile by first reacting a pyridinylmethyl lower-alkyl ketone with dimethylformamide di-(lower-alkyl) acetal to produce a 1-(pyridinyl)-2-(dimethylamino)ethenyl lower-alkyl ketone, then reacting said ketone with cyanoacetamide and acidifying the reaction mixture. The same process is disclosed in U.S. Patent 4,276,293.

U.S. Patent 4,223,149 discloses the process for preparing a 1,2-dihydro-2-oxo-5-(pyridinyl)nicotinonitrile by reacting alpha-(pyridinyl)-beta-[di-(loweralkyl)amino]-acrolein with malononitrile in a lower-alkanol.

U.S. Patent 2,824,121 discloses an improved process for preparing "oxy alkylidene compounds" using a weakly acid compound as catalytic agent, e.g., zinc chloride, the process being particularly useful in preparing ethoxymethylene malonic diethyl ester by reaction of diethyl malonate and triethyl orthoformate in the presence of acetic anhydride and catalytic amounts of zinc chloride.

Mezheritskii et al., Russian Chemical Reviews *42* (5), 392, 399—402 and 410 (1973), in a review article entitled "The Properties of Orthoesters and Their Applications in Organic Syntheses" has a section (pp. 399—402) "VIII. Reactions of Orthoesters with Substances Containing An Active Methylene Group". Shown *inter alia* is the reaction of diethyl malonate with triethyl orthoformate by heating the reactants in the presence of excess acetic anhydride to produce diethyl ethoxymethylenemalonate.

U.S. Patent No. 4,347,363 discloses the process for preparing 1,2-dihydro-6-methyl-2-oxo-5-(pyridinyl) nicotinonitriles by reacting a pyridinylmethyl methyl ketone with ethoxymethylenemalononitrile.

The present invention resides in the process for preparing cardiotonically active 1,2-dihydro-6-(lower-alkyl)-2-oxo-5-(pyridinyl)nicotinonitriles by first reacting a pyridinylmethyl lower-alkyl ketone with tri-(lower-alkyl) orthoformate, acetic anhydride and acetic acid to produce 2-(lower-alkoxy)-1-(pyridinyl)ethenyl lower-alkyl ketone and reacting said ketone with cyanoacetamide in the presence of a basic condensing agent and neutralizing the reaction mixture. The substituted nicotinonitriles produced as above and their cardiotonic use are disclosed in above U.S. Patent 4,313,951.

The invention includes a three-step process which comprises reacting pyridinylmethyl lower-alkyl ketone of the formula

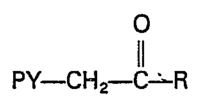

(I)

with tri-(lower-alkyl) orthoformate, acetic anhydride and acetic acid to produce 2-(lower-alkoxy)-1-(pyridinyl)-ethenyl lower-alkyl ketone of Formula II,

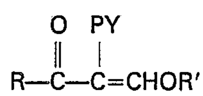

II

and reacting said ketone (II) with cyanoacetamide in the presence of a basic condensing agent and neutralizing the reaction mixture to produce 1,2-dihydro-6-R-2-oxo-5-PY-nicotinonitrile of Formula III,

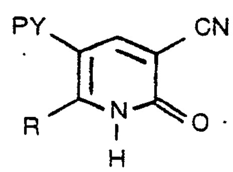

III

where R and R' are each ($C_1$—$C_4$)-alkyl and PY is 4- or 3-pyridinyl or 4- or 3-pyridinyl having one or two ($C_1$—$C_4$)-alkyl substituents.

In preferred embodiments 4(or 3)-pyridinylmethyl methyl or ethyl ketone is first reacted with triethyl or trimethyl orthoformate, acetic anhydride and acetic acid to produce 2-ethoxy(or methoxy)-1-(4- or 3-pyridinyl)ethenyl methyl (or ethyl) ketone, the latter ketone is next reacted with cyanoacetamide in the presence of at least a molar equivalent quantity of alkali hydroxide per mole of cyanoacetamide in a lower-alkanol and then the reaction mixture is neutralized to produce 1,2-dihydro-6-methyl (or ethyl)-2-oxo-5-[4(or 3)-pyridinyl]nicotinonitrile. In a particularly preferred embodiment 4-pyridinylmethyl methyl ketone is first reacted with triethyl orthoformate, acetic anhydride and acetic acid to produce 2-ethoxy-1-(4-pyridinyl)ethenyl methyl ketone, said latter ketone is then reacted with cyanoacetamide in the presence of at least a molar equivalent quantity of sodium hydroxide per mole of cyanoacetamide in methanol, and the reaction mixture is neutralized to produce 1,2-dihydro-6-methyl-2-oxo-5-(4-pyridinyl)nicotinonitrile.

The invention also includes a 2-(lower-alkoxy)-1-(pyridinyl)ethenyl lower-alkyl ketone having Formula II above or an acid-addition salt thereof, where R and R′ are each lower-alkyl and PY is 4- or 3-pyridinyl having one or two ($C_1$—$C_4$) alkyl substituents. Said ketone (II) is useful as an intermediate in the process of the invention given above. Preferred embodiments of this aspect of the invention are the ketones where R is methyl or ethyl, R′ is ethyl or methyl, and PY is 4-pyridinyl or 3-pyridinyl. A particularly preferred embodiment is the ketone (II) where R is methyl, R′ is ethyl and PY is 4-pyridinyl.

The term "lower-alkyl" as used herein, e.g., as the meaning for R in Formula I, II or III or for R′ in Formula II or as the meaning for one or two substituents for 4- or 3-pyridinyl, means alkyl radicals having from one to four carbon atoms which can be arranged as straight or branched chains, illustrated by methyl, ethyl, n-propyl, isopropyl, n-butyl, 2-butyl or isobutyl.

The term "lower-alkanol" as used herein, means alkanol having one to four carbon atoms which can be arranged as straight or branched chains, illustrated by methanol, ethanol, n-propanol, 2-propanol, n-butanol, 2-butanol or 2-methyl-n-propanol.

The term "PY" as used herein means 4- or 3-pyridinyl or 4- or 3-pyridinyl having one or two "lower-alkyl" substituents, illustrated by 2-methyl-4-pyridinyl, 3-methyl-4-pyridinyl, 2-methyl-3-pyridinyl, 2,6-dimethyl-4-pyridinyl, 6-methyl-3-pyridinyl (alternatively named 2-methyl-5-pyridinyl), 2,3-dimethyl-4-pyridinyl, 2-ethyl-4-pyridinyl, 2-isopropyl-4-pyridinyl, 2-n-butyl-4-pyridinyl, 2,6-diethyl-4-pyridinyl and 2,6-diisopropyl-4-pyridinyl.

The compounds of the invention having Formula II are useful both in the free base and in the form of acid-addition salts, and both forms are within the purview of the invention. The acid-addition salts are simply a more convenient form for use; and in practice, use of the salt form inherently amounts to use of the base form. The acids which can be used to prepare the acid-addition salts include preferably those which produce, when combined with the free base, pharmaceutically acceptable salts. In practicing the invention, it is convenient to use the free base form or the acetate salt thereof; however, other appropriate pharmaceutically acceptable salts within the scope of the invention are those derived from mineral acids such as hydrochloric acid, sulfuric acid, phosphoric acid and sulfamic acid; and organic acids such as citric acid, lactic acid, tartaric acid, methanesulfonic acid, ethanesulfonic acid, benzenesulfonic acid, p-toluenesulfonic acid, cyclohexylsulfamic acid and quinic acid giving the hydrochloride, sulfate, phosphate, sulfamate, citrate, lactate, tartrate, methanesulfonate, ethanesulfonate, benzenesulfonate, p-toluenesulfonate, cyclohexylsulfamate and quinate, respectively.

The acid-addition salts of said compounds of Formula II are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Although pharmaceutically acceptable salts of said compounds of Formula II are preferred, all acid-addition salts are within the scope of the invention. All acid-addition salts are useful as sources of the free base form even if the particular salt per se is desired only as an intermediate product as for example when the salt is formed only for purposes of purification or identification, or when it is used as an intermediate in preparing a pharmaceutically acceptable salt by ion exchange procedures.

The molecular structures of the products produced by the process of the invention were assigned on the basis of evidence provided by infrared, nuclear magnetic resonance and mass spectra, by chromatographic mobilities, and by the correspondence of calculated and found values for the elementary analyses for representative examples.

The manner of making and using the instant invention will now be generally described so as to enable a person skilled in the science of medicinal chemistry to make and use the same, as follows:

The process of the invention is carried out by mixing tri-(lower-alkyl) orthoformate, preferably triethyl or trimethyl orthoformate, with (pyridinyl)methyl lower-alkyl ketone (I) and acetic anhydride in acetic acid as solvent. After an exothermic reaction subsides, the reaction mixture is stirred at ambient temperature until completion of the reaction, as determined by tlc analysis, to produce 1-(lower-alkoxy)-2-(pyridinyl)ethenyl lower-alkyl ketone (II). The reaction is run using an excess each of tri-(lower-alkyl)orthoformate, preferably about 1.3 to 1.7 mole per mole of ketone, and acetic anhydride, preferably about 2.0 to 3.0 mole per mole of ketone. The resulting intermediate ketone (II) can be used in the next step of the process without further purification or if desired it can be isolated and further purified. The reaction of II with cyanoacetamide to produce 1,2-dihydro-6-(lower-alkyl)-2-oxo-5-(pyridinyl) nicotinonitrile (III, Q is CN) is carried out by heating the reactants in a suitable solvent in the presence of at least a molar equivalent of a basic condensing agent per mole of cyanoacetamide or malonamide and then neutralizing the reaction mixture. The reaction is preferably run using as basic condensing agent an alkali hydroxide, preferably sodium hydroxide, either in solid form or in concentrated aqueous solution, in a lower-alkanol, preferably methanol. Other basic condensing agents that can be used include alkali carbonates, e.g., sodium or potassium carbonate, lower-tertiary-amines such as tri-(lower-alkyl)-amines, e.g., trimethylamine, triethylamine, benzyldi-(lower-alkyl)amines, e.g., benzyldimethylamine, preferably in aqueous lower-alkanol. Still other basic condensing agents include sodium hydride, lithium diethylamide and lithium diisopropylamide which are used in an aprotic solvent, e.g., acetonitrile, benzene, ether, dioxane and tetrahydrofuran. The neutralization step is carried out by adding a suitable acid, e.g., acetic acid, to the alkaline reaction mixture, preferably to a pH of about 6 to 8.

Advantages of the instant process over the prior art process according to US—A—4313951 for preparing 1,2-dihydro-6-(lower-alkyl)-5-(pyridinyl)nicotinonitriles and corresponding substituted-nicotinamides reside in the utilization of less expensive and more readily available starting materials and in the easier production of a pharmaceutically pure product. For example, triethyl orthoformate is much less expensive than dimethylformamide dimethyl acetal and, further, it is much more readily available in the large quantities needed for large-scale or commercial production. Also, an unexpected advantage in the use of 1-(lower-alkoxy)-2-(4-pyridinyl)ethenyl lower-alkyl ketone (II) over the intermediate of the prior art, 1-dimethylamino-2-(4-pyridinyl)ethenyl lower-alkyl ketone, is the greater degree of regioselectivity in the addition of cyanoacetamide to 1-(lower-alkoxy)-2-(4-pyridinyl)ethenyl lower-alkyl ketone. Thus, reaction of cyanoacetamide with 1-ethoxy-2-(4-pyridinyl)ethenyl methyl ketone in place of the prior art intermediate 1-dimethylamino-2-(4-pyridinyl)ethenyl methyl ketone to prepare 1,2-dihydro-6-methyl-2-oxo-5-(4-pyridinyl)nicotinonitrile results in formation of less of the isomeric by-product, 1,2-dihydro-4-methyl-2-oxo-5-(4-pyridinyl)nicotinamide, thereby resulting in a simpler purification procedure required to produce a pharmaceutically pure compound.

Still additional advantages are afforded by the particularly preferred process of preparing 1,2-dihydro-6-methyl-2-oxo-5-(4-pyridinyl)nicotinonitrile by the two step process starting with 4-pyridinylmethyl methyl ketone in the first step to produce 1-(4-pyridinyl)ethenyl methyl ketone and then reacting said ketone in the second step with cyanoacetamide and at least a molar equivalent quantity of sodium hydroxide (either in solid form or in concentrated aqueous solution) in methanol to produce said nicotinonitrile. Thus, the two-step preparation can be conveniently run in one pot without isolating the intermediate 1-(4-pyridinyl)ethenyl methyl ketone. Moreover, the process is more economical in using the inexpensive sodium hydroxide as condensing agent. Also, it eliminates the need to recrystallize the said nicotinonitrile final product from dimethylformamide; and, the final product, i.e., 1,2-dihydro-6-methyl-2-oxo-5-(4-pyridinyl)nicotinonitrile, is obtained in an even purer form and in higher yield.

The following examples will further illustrate the invention.

A. 1-(Lower-alkoxy)-2-(pyridinyl)ethenyl lower-alkyl ketones

A—1. *1-Ethoxy-2-(4-pyridinyl)ethenyl Methyl Ketone,* alternatively named 4-ethoxy-3-(4-pyridinyl)-3-buten-2-one — A 135 g portion (1 m.) of 1-(4-pyridinyl)propan-2-one was added to 249 ml (1.5 m.) of triethyl orthoformate and 259 ml (2.75 m.) acetic anhydride in 250 ml acetic acid in a 3-neck 2 l. flask. A mild exotherm ensued which carried the temperature from 21°C. to a peak of 56°C. after 30 minutes. The reaction mixture was stirred at room temperature for about 12 to 15 hours or for sufficient time to effect complete reaction as determined by tlc on silica gel with a 3:1 mixture of chloroform/methanol as the mobile phase. The volatiles were removed by distillation under reduced pressure (water aspirator) using a pot temperature of 80°C. Ethanol (200 ml) was added followed by further distillation *in vacuo* to a pot temperature of 95°C. in order to remove the excess acetic anhydride. (Methanol could be used in place of ethanol.) The residue, 177.8 g deep red oil, consisting primarily of 1-ethoxy-2-(4-pyridinyl)ethenyl methyl ketone, was used directly in the next step (Example B—1) without further purification.

A—2. *1-Ethoxy-2-(4-pyridinyl)ethenyl Methyl Ketone* — To a 104.5 g portion (0.77 m.) of 1-(4-pyridinyl) propan-2-one were successively added 141 ml (0.85 m.) of triethylorthoformate, 175 ml (1.86 m.) of acetic anhydride and 200 ml of acetic acid whereupon a exothermic reaction ensued raising the reaction temperature from 23°C. to 49°C. over a 30 minute period. The reaction mixture was then stirred at ambient temperature for about 16 hours and then heated *in vacuo* to remove the volatile materials. The residue was dissolved in ethyl acetate and the mixture treated with saturated sodium bicarbonate solution until foaming ceased. The organic layer was separated, dried over anhydrous sodium sulfate, treated with decolorizing charcoal and filtered. The filtrate was heated *in vacuo* to remove the solvent and the residue was swirled with about 300 ml of ether for about 5 minutes whereupon crystallization resulted. The mixture was stirred in an ice bath and the separated solid was collected, washed with cold ether and dried at 45°C. to yield 78.5 g of orange solid. The solid was recrystallized from 200 ml of isopropyl acetate using decolorizing charcoal (5 g). The filtrate obtained after removal of the decolorizing charcoal was cooled whereupon the solid separated. The solid was collected, washed with cold ether and dried in a vacuum oven at 45°C. overnight to yield 63 g of 1-ethoxy-2-(4-pyridinyl)ethenyl methyl ketone, m.p. 84—88°C.

Acid-addition salts of 1-ethoxy-2-(4-pyridinyl)ethenyl methyl ketone are conveniently prepared by adding to a mixture of 1 g of 1-ethoxy-2-(4-pyridinyl)ethenylmethyl ketone in about 20 ml of methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively.

Following the procedure described in Example A—2 but using in place of triethyl orthoformate a molar equivalent quantity of trimethyl orthoformate or tri-n-propyl orthoformate, it is contemplated that the compounds of Examples A—3 and A—4 can be prepared.

A—3. 1-Methoxy-2-(4-pyridinyl)ethenyl methyl ketone.

A—4. 1-n-Propoxy-2-(4-pyridinyl)ethenyl methyl ketone.

Following the procedure described in Example A—2 but using in place of 1-(4-pyridinyl)propan-2-one a molar equivalent quantity of the appropriate (pyridinyl)methyl lower-alkyl ketone, it is contemplated that there can be obtained the corresponding 1-ethoxy-2-(pyridinyl)ethenyl lower-alkyl ketones of Examples A—5 through A—10.

A—5. 1-Ethoxy-2-(3-pyridinyl)ethenyl methyl ketone, using 1-(3-pyridinyl)propan-2-one.

A—6. 1-Ethoxy-2-(2-methyl-4-pyridinyl)ethenyl methyl ketone, using 1-(2-methyl-4-pyridinyl)propan-2-one.

A—7. 1-Ethoxy-2-(2-ethyl-4-pyridinyl)ethenyl methyl ketone, using 1-(2-ethyl-4-pyridinyl)propan-2-one.

A—8. 1-Ethoxy-2-(2,6-dimethyl-4-pyridinyl)ethenyl methyl ketone, using 1-(2,6-dimethyl-4-pyridinyl)propan-2-one.

A—9. 1-Ethoxy-2-(4-pyridinyl)ethenyl ethyl ketone, using 1-(4-pyridinyl)butan-2-one.

A—10. 1-Ethoxy-2-(4-pyridinyl)ethenyl n-propyl ketone, using 1-(4-pyridinyl)pentan-2-one.

A—11. *1-Ethoxy-2-(4-pyridinyl)ethenyl Ethyl Ketone*, 113 g of a dark red oil consisting primarily of said ketone, was prepared following the procedure described in Example A—1 but using 74.5 g of 1-(4-pyridinyl)butan-2-one, 125 ml of triethyl orthoformate, 130 ml of acetic anhydride and 125 ml of glacial acetic acid. The product was used in the next step (Example C—8) without further purification.

B. 1,2-Dihydro-6-(lower-alkyl)-2-oxo-5-(pyridinyl)nicotinonitriles

B—1. *1,2-Dihydro-6-methyl-2-oxo-5-(4-pyridinyl)nicotinonitrile* — Sodium methylate (189 g 3.5 m.) was added to 2 l. of dry methanol (temperature 60°C.); 105 g (1.25 m.) of cyanoacetamide was added to one portion followed immediately by a solution of 177.8 g (about 1 m.) of 1-ethoxy-2-(4-pyridinyl)ethenyl methyl ketone (from Example A—1) in 500 ml methanol which was also added as quickly as feasible (temperature 55°C.). The reaction mixture was brought to reflux during which time the product began to separate, initially as an almost unstirrable gel; however, upon reaching reflux, the product crystallized into an easily stirred but thick slurry. Refluxing was continued for 1 hour. The reaction was cooled to 55°C. and treated carefully over 5 minutes with 150 ml (2.62 m.) glacial acetic acid (temperature 63°C.). 2 l. Methanol was distilled off at atmospheric pressure leaving a thick paste. To the paste was added 2 l. cold water and the pH was adjusted to 6.5—7.0 with 10 ml acetic acid. The reaction mixture was cooled to <5°C. The solid was collected on a funnel, washed with 2 × 500 ml. cold water, pressed dry and drying continued in a vacuum chamber at 65°C. overnight to yield 147.5 g of a pinkish-tan solid. A 42.2 g portion of this solid was added to 100 ml 8% aqueous NaOH at room temperature. The mixture was stirred until dissolved (5 min.), treated with 4 g of decolorizing charcoal and stirred an additional 5 minutes. The resultant mixture was filtered through a finely divided filter-aid, e.g., cellulose based filter aid (SOLKA FLOC®) or diatomaceous earth. The filtercake was washed with 2 × 25 ml water and the filtrates combined. The filtrates were treated with 12 ml glacial acetic acid to pH 6.5—7.0. The thick slurry was heated at 90°C. with stirring for 15 minutes to digest (optional). Cooling to <10°C., filtration and water wash with 2 × 50 ml water affords a pale pink solid. The material was dried *in vacuo* at 65°C. overnight to yield 41 g pale pink solid (97% recovery). A 37.5 g portion of this solid was dissolved by heating with 200 ml of dimethylformamide, the solution was allowed to cool with stirring to room temperature, cooled in ice and the product was filtered, washed with 2 × 100 ml ethyl acetate, dried *in vacuo* at 65°C. overnight to yield as an off-white solid, 34 g (91% recovery) of 1,2-dihydro-6-methyl-2-oxo-5-(4-pyridinyl)nicotinonitrile, m.p. >300°C.

Following the procedure described in Example B—1 but using in place of 1-ethoxy-2-(4-pyridinyl)ethenyl methyl ketone a molar equivalent quantity of the appropriate 1-alkoxy-2-(pyridinyl)ethenyl lower-alkyl ketone, it is contemplated that there can be obtained the corresponding 1,2-dihydro-6-(lower-alkyl)-2-oxo-5-(pyridinyl)-nicotinonitriles of Examples C—2 through C—7.

B—2. 1,2-Dihydro-6-methyl-2-oxo-5-(3-pyridinyl)-nicotinonitrile, using 1-ethoxy-2-(3-pyridinyl)ethenyl methyl ketone.

B—3. 1,2-Dihydro-6-methyl-5-(2-methyl-4-pyridinyl)-2-oxonicotinonitrile, using 1-ethoxy-2-(2-methyl-4-pyridinyl)ethenyl methyl ketone.

B—4. 5-(2-Ethyl-4-pyridinyl)-1,2-dihydro-6-methyl-2-oxonicotinonitrile, using 1-ethoxy-2-(2-ethyl-4-pyridinyl)ethenyl methyl ketone.

B—5. 1,2-Dihydro-6-methyl-5-(2,6-dimethyl-4-pyridinyl)-2-oxonicotinonitrile, using 1-ethoxy-2-(2,6-dimethyl-4-pyridinyl)ethenyl methyl ketone.

B—6. 1,2-Dihydro-6-n-propyl-5-(4-pyridinyl)-2-oxonicotinonitrile, using 1-ethoxy-2-(4-pyridinyl)ethenyl n-propyl ketone.

B—7. 1,2-Dihydro-6-methyl-2-oxo-5-(4-pyridinyl)nicotinonitrile, using 1-methoxy-2-(4-pyridinyl)ethenyl methyl ketone or 1-(n-propoxy)-2-(4-pyridinyl)ethenyl methyl ketone.

B—8. *6-Ethyl-1,2-dihydro-5-(4-pyridinyl)-2-oxonicotinonitrile*, 92 g, m.p. >300°C., was prepared following the procedure described in Example C—1 but using 95 g (1.75 m.) of sodium methoxide, 1 liter of dry methanol, 52.5 g (0.625 m.) of cyanoacetamide, 113 g (0.5 m.) of 1-ethoxy-2-(4-pyridinyl)ethenyl ethyl ketone, a reflux period of one hour, adding 75 ml of glacial acetic acid to the warm reaction mixture, distilling off 1 l. of methanol, adding 1 l. of water, adjusting the pH to 6.5 by adding acetic acid, cooling the mixture in an ice bath, collecting the precipitated product, washing it successively with a little ethanol and then ether, and drying it in a vacuum chamber at 65°C.

B—9. *1,2-Dihydro-6-methyl-2-oxo-5-(4-pyridinyl)nicotinonitrile* — A 30 gallon glass-lined kettle was charged while stirring with 11.2 kg of (186.4 moles) of acetic acid, 12.3 kg (120.6 moles) of acetic anhydride, and 7.32 kg (49.5 moles) of triethyl orthoformate to obtain a clear, colorless solution at 19°C. To the solution was added with stirring 5.24 kg (38.0 moles) of 1-(4-pyridinyl)-2-propanone and a mild exotherm was monitored. After 1 1/2 hour very gentle cooling was applied to maintain a maximum temperature of 50°C.

until the exotherm had subsided (1 3/4 hr.). Stirring at ambient temperature for 17 hours gave a red-orange solution (26°C. final temperature), which was concentrated *in vacuo* using 60—70°C. water in the kettle jacket until the reaction volume was reduced to a volume of about 11—12 liters (required 2 hrs.). The concentrate, which contained in solution 1-ethoxy-2-(4-pyridinyl)ethenyl methyl ketone in the form of its acetate salt, was cautiously diluted with 52 l. of methanol while cooling to 20°C. 2-Cyanoacetamide (4.48 kg, 53.3 moles) was then added, followed by 6.1 l. of 35% aqueous sodium hydroxide solution to reach pH 12. An additional 4.42 l. of 35% aqueous sodium hydroxide solution (53.3 moles) was added and the mixture was heated to reflux for 1 hour (pot temp. 70°C.). After cooling to 50°C., the pH was adjusted to 6.0—6.3 by the addition of 4.6 l. of acetic acid. The mixture was cooled to 0°C. for 15 minutes and filtered on a 28" diameter ceramic vacuum filter. The filter cake was washed with 7.6 l. of cold methanol and then 19 l. of deionized water. It was dried *in vacuo* at 60°C. affording, as a pink powder, 6.38 kg of 1,2-dihydro-6-methyl-2-oxo-5-(4-pyridinyl)nicotinonitrile (79.4% yield). This product was 99.0—99.5% pure as estimated by tlc. A second crop of 323 g (4% of theoretical yield) was obtained by concentrating the filtrate in vacuo to about one-half the volume, diluting to the original volume with water, then reconcentrating *in vacuo* to 3/4th volume, and collecting the product. A 6.36 kg portion of the above 6.38 kg of pink powder was further purified as follows. A 30 gallon glass-lined kettle was charged with 17.5 l. of deionized water and 2.81 l. of 35% aqueous sodium hydroxide solution. The 6.36 kg of product was added and the resulting solution was treated at ambient temperature for 15 minutes with 670 g of charcoal. The charcoal was removed by filtration through a finely divided filter-aid, e.g., cellulose based filter aid (SOLKA FLOC®) or diatomaceous earth on an 18" ceramic vacuum filter and the cake was washed with 7.65 l. of deionized water. The filtrate and wash were combined and slowly (over 1/2 hour) treated with acetic acid (2.16 l. required) to pH 6.5. After stirring at ambient temperature for 45 minutes, the product was collected on a 28" ceramic filter and washed with 15 l. of deionized water. Drying *in vacuo* at 60°C. overnight gave 6.22 kg of off-white product, 1,2-dihydro-6-methyl-2-oxo-5-(4-pyridinyl)nicotinonitrile, m.p. >300°C., (6.22/6.36 = 97.8% recovery).

B—10. *1,2-Dihydro-6-methyl-2-oxo-5-(4-pyridinyl)nicotinonitrile* — To a solution containing 2.6 g of sodium hydroxide pellets dissolved in 41 ml of methanol was added 2.5 g of cyanoacetamide with stirring. After dissolution was complete (about 5 minutes), to the solution was added a solution containing 5.0 g of 1-ethoxy-2-(4-pyridinyl)-ethenyl methyl ketone in 15 ml of methanol and the reaction mixture was heated to reflux for one hour, cooled to 50°C. and treated with 4.2 ml of acetic acid. The methanol was distilled off at atmospheric pressure and the residue treated with 55 ml of water. Since the pH was 6.0, there was no need to adjust it. The mixture was cooled to 5°C. and the separated solid was collected, washed successively with 50 ml portions of cold water, ethanol and ether, and air-dried to produce 4.7 g (85.5% yield) of 1,2-dihydro-6-methyl-2-oxo-5-(4-pyridinyl)nicotinonitrile, m.p. >300°C.

Following the procedure described in Example B—10 but using in place of 1-ethoxy-2-(4-pyridinyl)ethenyl methyl ketone a molar equivalent quantity of the appropriate 1-alkoxy-2-(pyridinyl)ethenyl lower-alkyl ketone, it is contemplated that there can be obtained the corresponding 1,2-dihydro-6-(lower-alkyl)-2-oxo-5-(pyridinyl)-nicotinonitriles of Examples B—11 through B—16.

B—11. 1,2-Dihydro-6-methyl-2-oxo-5-(3-pyridinyl)-nicotinonitrile, using 1-ethoxy-2-(3-pyridinyl)ethenyl methyl ketone.

B—12. 1,2-Dihydro-6-methyl-5-(2-methyl-4-pyridinyl)-2-oxonicotinonitrile, using 1-ethoxy-2-(2-methyl-4-pyridinyl)ethenyl methyl ketone.

B—13. 5-(2-Ethyl-4-pyridinyl)-1,2-dihydro-6-methyl-2-oxonicotinonitrile, using 1-ethoxy-2-(2-ethyl-4-pyridinyl)ethenyl methyl ketone.

B—14. 1,2-Dihydro-6-methyl-5-(2,6-dimethyl-4-pyridinyl)-2-oxonicotinonitrile, using 1-ethoxy-2-(2,6-dimethyl-4-pyridinyl)ethenyl methyl ketone.

B—15. 1,2-Dihydro-6-n-propyl-5-(4-pyridinyl)-2-oxonicotinonitrile, using 1-ethoxy-2-(4-pyridinyl)ethenyl n-propyl ketone.

B—16. 1,2-Dihydro-6-methyl-2-oxo-5-(4-pyridinyl)-nicotinonitrile, using 1-methoxy-2-(4-pyridinyl)ethenyl methyl ketone or 1-(n-propoxy)-2-(4-pyridinyl)ethenyl methyl ketone.

## Claims

1. A 2-(lower-alkoxy)-1-(pyridinyl)ethenyl lower-alkyl ketone of the Formula II

$$R-\overset{\overset{O}{\|}}{C}-\overset{\overset{PY}{|}}{C}=CHOR' \qquad II$$

or an acid-addition salt thereof, where R and R′ are each $(C_1—C_4)$ alkyl and PY is 4- or 3-pyridinyl or 4- or 3-pyridinyl having one or two $(C_1—C_4)$ alkyl substituents.

2. A compound according to claim 1, wherein R is methyl or ethyl, R′ is ethyl or methyl, and PY is 4-pyridinyl or 3-pyridinyl.

3. 2-Ethoxy-1-(4-pyridinyl)ethenyl methyl ketone according to claim 1.

4. 2-Ethoxy-1-(4-pyridinyl)ethenyl ethyl ketone according to claim 1.

5. A process for the preparation of a compound of Formula II according to claim 1, which comprises reacting a pyridinyl-methyl lower-alkyl ketone of the Formula I

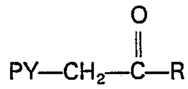

I

wherein R and PY are as defined in claim 1, with tri($C_1$—$C_4$-alkyl) orthoformate, acetic anhydride and acetic acid.

6. A process according to claim 5, wherein said lower alkyl in said ketone and orthoformate is methyl or ethyl.

7. A process for the preparation of a compound of the Formula III

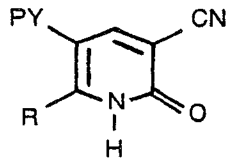

III

wherein R and R′ are each $C_1$—$C_4$ alkyl and PY is 4- or 3-pyridinyl or 4- or 3-pyridinyl having one or two ($C_1$—$C_4$)alkyl substituents
which comprises reacting a compound of the Formula II according to claim 1 wherein R, PY and R′ are as defined in claim 1 with cyanoacetamide in the presence of a basic condensing agent and neutralizing the reaction mixture.

8. A process according to claim 7, wherein said basic condensing agent is an alkali hydroxide in a lower alkanol, e.g. methanol.

9. A process according to claim 8, wherein at least a molar equivalent quantity of alkali hydroxide per mole of cyanoacetamide is present.

**Patentansprüche**

1. 2-(Nied.Alkoxy)-1-(pyridinyl)ethenyl-nied.alkylketon der Formel II

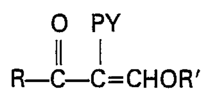

II

oder Säureadditionssalz derselben, wobei R und R′ jeweils ein ($C_1$—$C_4$)-Alkyl und PY ein 4- oder 3-Pyridinyl oder ein 4- oder 3-Pyridinyl mit einem oder 2 ($C_1$—$C_4$)-Alkylsubstituenten ist.

2. Verbindung nach Anspruch 1, dadurch gekennzeichnet, daß R Methyl oder Ethyl ist, R′ Ethyl oder Methyl ist und PY 4-Pyridinyl oder 3-Pyridinyl ist.

3. 2-Ethoxy-1-(4-pyridinyl)ethenylmethylketon nach Anspruch 1.

4. 2-Ethoxy-1-(4-pyridinyl)ethenylethylketon nach Anspruch 1.

5. Verfahren zum Erzeugen einer Verbindung der Formel II nach Anspruch 1, dadurch gekennzeichnet, daß ein Pyridinylmethyl-nied.alkylketon der Formel I

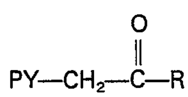

I

in der R und RY die im Anspruch 1 angegebenen Bedeutungen haben, mit Tri($C_1$—$C_4$-alkyl)orthoformiat, Essigsäureanhydrid und Essigsäure umgesetzt wird.

6. Verfahren nach Anspruch 5, dadurch gekennzeichnet, daß das niedere Alkyl in dem Keton oder dem Orthoformiat Methyl oder Ethyl ist.

7. Verfahren zum Erzeugen einer Verbindung der Formel III

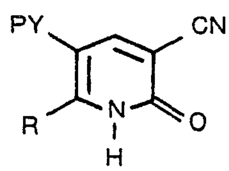

III

wobei R und R′ jeweils ein ($C_1$—$C_4$)-Alkyl und PY ein 4- oder 3-Pyridinyl oder ein 4- oder 3-Pyridinyl mit einem oder 2 ($C_1$—$C_4$)-Alkylsubstituenten ist,

wobei eine Verbindung der Formel II nach Anspruch 1, in der R, PY und R' die im Anspruch 1 angegebenen Bedeutungen haben, in Gegenwart eines basischen Kondensationsmittels mit Cyanoacetamid umgesetzt und das Reaktionsgemisch neutralisiert wird.

8. Verfahren nach Anspruch 7, dadurch gekennzeichnet, daß das basische Kondensationsmittel ein Alkalihydroxid in einem niederen Alkanol, z.B. Methanol, ist.

9. Verfahren nach Anspruch 8, dadurch gekennzeichnet, daß die Menge des Alkalihydroxids mindestens einem Moläquivalent pro Mol Cyanoacetamid entspricht.

**Revendications**

1. 2-(alcoxy inférieur)-1-(pyridinyl)éthényl-(alkyle inférieur)cétone de formule II

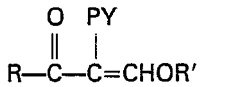

II

dans laquelle R et R' sont chacun un groupe alkyle en $C_1$—$C_4$ et PY est le radical 4- ou 3-pyridinyle ou un radical 4- ou 3-pyridinyle comportant un ou deux substituants alkyle en $C_1$—$C_4$, ou sel d'addition d'acide de celle-ci.

2. Composé selon la revendication 1, dans lequel R est le groupe méthyle ou éthyle, R' est le groupe éthyle ou méthyle, et PY est le radical 4-pyridinyle ou 3-pyridinyle.

3. 2-éthoxy-1-(4-pyridinyl)éthényl-méthyl-cétone selon la revendication 1.

4. 2-éthoxy-1-(4-pyridinyl)éthényl-éthyl-cétone selon la revendication 1.

5. Procédé pour la préparation d'un composé de formule II selon la revendication 1, qui comprend la réaction d'une pyridinyl-méthyl-(alkyle inférieur)cétone de formule I

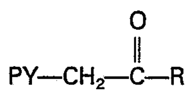

I

dans laquelle R et PY sont tels que définis dans la revendication 1, avec un orthoformiate de tri(alkyle en $C_1$—$C_4$), l'anhydride acétique et l'acide acétique.

6. Procédé selon la revendication 5, dans lequel ledit alkyle inférieur dans ladite cétone et dans ledit orthoformiate est le groupe méthyle ou éthyle.

7. Procédé pour la préparation d'un composé de formule III

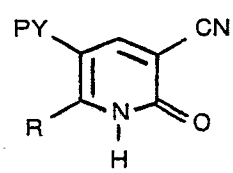

III

dans laquelle R et R' sont chacun un groupe alkyle en $C_1$—$C_4$ et PY est le radical 4- ou 3-pyridinyle ou un radical 4- ou 3-pyridinyle comportant un ou deux substituants alkyle en $C_1$—$C_4$,
qui comprend la réaction d'un composé de formule II selon la revendication 1, dans laquelle R, PY et R' sont tels que définis dans la revendication 1, avec le cyano-acétamide en présence d'un agent de condensation basique, et la neutralisation du mélange réactionnel.

8. Procédé selon la revendication 7, dans lequel ledit agent de condensation basique est un hydroxyde alcalin dans un alcanol inférieur, le méthanol par exemple.

9. Procédé selon la revendication 8, dans lequel est présent au moins un équivalent molaire d'hydroxyde alcalin par mole de cyano-acétamide.